# EUROPEAN PATENT SPECIFICATION

(11) **EP 4 257 057 B1**
(45) Date of publication and mention of the grant of the patent: **10.09.2025**
(21) Application number: 23166496.2
(22) Date of filing: 04.04.2023
(51) Int. Cl.: A61B 8/08, A61B 5/00, A61B 8/00

(54) **SYSTEM FOR SCREENING TISSUE ON THE PRESENCE OF MALIGNANT CELLS**
SYSTEM ZUM SCREENING VON GEWEBE AUF DER BASIS MALIGNER ZELLEN
SYSTÈME DE CRIBLAGE DE TISSU SUR LA PRÉSENCE DE CELLULES MALIGNES

(30) Priority: 04.04.2022 NL 2031483
(43) Date of publication of application: 11.10.2023
(73) Proprietor: Teledyne Dalsa B.V., 5656 AE Eindhoven (NL)
(72) Inventor: DEN HEETEN, Gerard Johan, Enschede (NL); AARNINKHOF, Ivo Jan Christian, Enschede (NL)
(74) Representative: HGF

(56) References cited:
- US-A1- 2014 288 424
- YAMAKOSHI YOSHIKI ET AL: "Shear Wave Imaging of Breast Tissue by Color Doppler Shear Wave Elastography", IEEE TRANSACTIONS ON ULTRASONICS, FERROELECTRICS, AND FREQUENCY CONTROL, IEEE, USA, vol. 64, no. 2, 2 February 2017 (2017-02-02), pages 340 - 348, XP011640314, ISSN: 0885-3010, [retrieved on 20170202], DOI: 10.1109/TUFFC.2016.2626359
- CAROLINA AMADOR ET AL: "Shearwave dispersion ultrasound vibrometry (sduv) on swine kidney", IEEE TRANSACTIONS ON ULTRASONICS, FERROELECTRICS, AND FREQUENCY CONTROL, IEEE, USA, vol. 58, no. 12, 2 December 2011 (2011-12-02), pages 2608 - 2619, XP011402204, ISSN: 0885-3010, DOI: 10.1109/TUFFC.2011.2124
- STEPHEN A BOPPART ET AL: "COHERENCE IMAGING TECHNOLOGIES FOR THE MEASUREMENT OF TISSUE AND CELL BIOMECHANICS", 2 January 2010 (2010-01-02), XP055333440, Retrieved from the Internet <URL:https://www.ideals.illinois.edu/bitstream/handle/2142/16859/1_Liang_Xing.pdf?sequence=3&isAllowed=y> [retrieved on 20170109]

## Description

The invention relates to a system for screening tissue on the presence of malignant cells in said tissue, which system comprises a wave detector and a data processing device connected or connectable to the wave detector for processing data received from the wave detector. This is what the invention has in common with the prior art.

Although the invention is not restricted thereto, examples of screening tissue on the presence of malignant cells can in particular be found in the field of mammography. Examples of mammography devices and methods for processing mammography images are for instance provided by US2012/033786 (Zinreich, US2015/139523 (EBM Technologies), US2021/315533 (Vieworks Co Ltd), JP2014036906 (Toshiba), KR20150070722 (Samsung), US2010/104063 (Gen. Electric), CN 110364250 (University of Shenshen) and JP2016106963 (Canon), just to name a few of the 15,320 searchable hits on the topic of 'mammography' provided on espacenet.com. On 'detection of malignant cells or tissue' in general there are even more than 74,000 searchable hits. In other words the skilled person in the field of tissue screening in search of malignant cells is overloaded with information how this can be accomplished.

Other background disclosures are provided by:
i) Yamakoshi Yoshiki et al "Shear Wave Imaging of Breast Tissue by Color Doppler Shear Wave Elastography", IEEE Transactions on Ultrasonics, Ferroelectrics and Frequency Control, IEEE, USE vol. 64, no. 2, 2 February 2017 (2017-02-02), pages 340-348;
ii) CAROLINA AMADOR ET AL: "Shearwave dispersion ultrasound vibrometry (sduv) on swine kidney", IEEE TRANSACTIONS ON ULTRASONICS, FERROELECTRICS, AND FREQUENCY CONTROL, IEEE, USA, vol. 58, no. 12, 2 December 2011 (2011-12-02), pages 2608-2619;
iii) Stephen A Boppart ET AL: "COHERENCE IMAGING TECHNOLOGIES FOR THE MEASUREMENT OF TISSUE AND CELL BIOMECHANICS", 2 January 2010 (2010-01-02); and
iv) US 2014/288424 A1.

A prominent disadvantage of prior art systems and methods is that they are either invasive, or require educated and trained personnel to carry out those prior art methods, or are unpleasant for the patient, or subject the patient to for instance x-ray imaging which in itself may pose health risks.

### SUMMARY OF THE INVENTION

It is an object of the invention to propose a system in which these disadvantages are at least in part alleviated or mitigated.

According to the invention a system is proposed with the features of one or more of the appended claims.

### SUMMARY OF THE DISCLOSURE

In a first aspect of the disclosure the system comprises an actuator to mechanically excite the tissue which is suspected to comprise malignant cells, and the data processing device comprises an analyzer connected to the wave detector for analyzing the data received from the wave detector in response to the actuator mechanically exciting the tissue, which analyzer is arranged to identify the tissue with an elevated probability to comprise malignant cells in comparison with tissue that is not suspected to comprise malignant cells.

According to a first aspect of the present disclosure there is provide a system for screening tissue on the presence of malignant cells in said tissue, which system comprises a wave detector and a data processing device connected or connectable to the wave detector for processing data received from the wave detector. The system comprises an actuator to mechanically excite the tissue which is suspected to comprise malignant cells, and the data processing device comprises an analyzer connected to the wave detector for analyzing the data received from the wave detector in response to the actuator mechanically exciting the tissue, which analyzer is arranged to identify the tissue with an elevated probability to comprise malignant cells in comparison with tissue that is not suspected to comprise malignant cells.

The analyzer may determine from the data received from the wave detector an estimated stiffness value of the tissue under investigation, wherein the analyzer is arranged to use this stiffness value as an indication for the presence or nonpresence of malignant cells.

The analyzer may determine from the data received from the wave detector local differences of estimated stiffness values of the tissue under investigation, wherein the analyzer is arranged to use these local differences of stiffness values as an indication for the presence or nonpresence of malignant cells.

The data processing device may comprise or connect to a data storage device for storage of data received from the wave detector.

**The** analyzer may be equipped to operate on data received from the wave detector and/or from the data storage device, wherein the data has different timestamps so as to enable a longitudinal comparison of data over time and to derive from this comparison an indication of the probable presence of malignant cells.

The actuator may be one of a sound emitter, an ultrasound emitter, a pulsed laser source, a displacement actuator.

The actuator is a displacement actuator for displacing a tissue engaging surface along a predetermined path and/or applying a predetermined load on the tissue to be investigated.

The wave detector may be arranged to collect and provide data to the data processing device, said data relating at least to the tissue being disengaged and released from a load by the displacement actuator, and to the tissue being engaged and loaded by the displacement actuator to a predetermined full load.

The wave detector may be at least one of an (ultra-) sound wave detector, a visual light spectrum 2D or 3D static picture camera, a visual light spectrum 2D or 3D moving picture camera, an infrared detector, an ultraviolet detector, a LIDAR, a radar, a microwave antenna.

The camera may be a CCD-camera.

The displacement actuator is equipped with at least one marker which enables position detection of the tissue engaging surface or contact area of the displacement actuator.

A further detectable marker may be provided on the supporting surface against which the displacement actuator presses the tissue under investigation.

The wave detector may be arranged to detect the position of the marker.

The data collected and provided by the wave to the data processing device may relate to a complete load-path-trajectory wherein the load provided by the displacement actuator on the tissue develops from no-load to the predetermined full load.

The data processing device may be equipped to estimate from the data a contact area between the displacement actuator and the tissue during engagement of the tissue by the displacement actuator.

The displacement actuator may be equipped with a capacitive measurement organ to determine a contact area between the displacement actuator and the tissue during engagement of the tissue by the displacement actuator.

**The** analyzer may be arranged to derive an estimated stiffness value of the tissue from the estimated or measured contact area between the displacement actuator and the tissue during engagement of the tissue by the displacement actuator, or from a longitudinal comparison of the estimated or measured contact area developing over time and to derive from this comparison an indication of the presence of malignant cells.

The estimation or measurement of the contact area between the displacement actuator and the tissue may be monitored during a complete cycle of engaging and disengaging of the tissue by the displacement actuator.

The analyzer may be arranged to derive an estimated stiffness value of the tissue from a measured or calculated force applied to the tissue by the displacement actuator and the estimated or measured contact area between the displacement actuator and the tissue during engagement of the tissue by the displacement actuator.

The analyzer may be arranged to derive an estimated stiffness value of the tissue from a longitudinal comparison of a measured or calculated force applied to the tissue by the displacement actuator over time, and the estimated or measured contact area developing over time between the displacement actuator and the tissue during engagement of the tissue by the displacement actuator.

The data processing device may be equipped to determine from the data an estimated volume of the tissue being investigated.

The data processing device may be equipped to determine from the data an estimated volume of the tissue being investigated when the displacement actuator is inactive and/or when the displacement actuator applies a load on the tissue and/or during a complete cycle of engaging and disengaging of the tissue by the displacement actuator.

The analyzer may be arranged to derive an estimated stiffness value of the tissue from a measured or calculated force applied to the tissue and at least one of the estimated volume of the tissue, the contact area between the displacement actuator and the tissue, and a projected area obtained from a projection of the volume of the tissue, one thing and another derived from the data when the displacement actuator is inactive and/or when the displacement actuator applies a constant load to the tissue, and/or from the data when the displacement actuator applies an increasing load on the tissue.

**The** system may be equipped with a position sensor for measuring a position of a tissue engaging surface or contact area of the displacement actuator and/or a force sensor for measuring a force that the displacement actuator provides to the tissue, which position sensor and/or force sensor are connected to the data processing device for use by the data processing device in combination with the estimated or measured contact area between the displacement actuator and the tissue during engagement of the tissue by the displacement actuator so as to arrange that the load applied by the displacement actuator to the tissue is normalized to a pressure which the displacement actuator applies to the tissue.

The system may be designed for processing data derived from a humans breast.

The system may be designed for processing data derived from a left breast and a right breast from a same patient, and arranging the analyzer to derive from differences between the left breast and the right breast an indication of malignant cells in one breast or both breasts.

The systems and methods disclosed herein may be based on the thought that developing tumors can already be felt or seen, or measured by ultrasound for instance, although in conventional screening it is more common to apply imaging modalities. The system and method of the invention can thus to a certain extent narrow the gap between a physical examination and imaging. A further benefit is that the invention provides the system with the capability (but not the necessity) to automatically identify tissue with malignant cells, which opens the possibility (but again: not the necessity) to apply the method and at least part of the system in the privacy of one's own home, while the system entertains a remote connection between the (local) wave detector and the (remotely positioned) data processing device.

The term 'wave or waves' is used to express the generality of the type of detector and in connection therewith the type of actuator that may be used. It is for instance proposed that the actuator is one of a sound emitter, an ultrasound emitter, a pulsed laser source (which inflicts ultrasound waves in the tissue), or simply a displacement actuator. On the other hand the detector is preferably one of an (ultra-)sound wave detector, a visual light spectrum 2D or 3D static picture camera, a visual light spectrum 2D or 3D moving picture camera, an infrared detector, an ultraviolet detector, but it can also be a LIDAR, a radar, or a microwave antenna. When using a camera, which can be regarded as an electromagnetic wave detector, it is preferred that this is a CCD camera, which makes digital processing of data from the camera received by the data processing device easy.

Since the singular embraces the plural, it is evident that there may also be more than one detector, or even detectors of different design and functionality. To stipulate that all types of detector may be used, the following discussion will consistently refer to 'detector' or 'wave detector', which may then be understood as any one of the indicated types of detector.

**An** important aspect of the system of the disclosure is that the analyzer determines from the data received from the wave detector an estimated stiffness value of the tissue under investigation, wherein the analyzer is arranged to use this stiffness value as an indication for the presence or nonpresence of malignant cells. This is based on the view of the inventors that the presence of malignant cells in certain tissue also leads to changed mechanical properties of such tissue, and that monitoring these mechanical properties makes possible to accomplish potentially a more early detection of the malignant cells in a significant amount of cases where prior art modalities fail. In such prior art screening modalities this information is not used.

In a certain example of the system of the disclosure it is desirable to fine-tune the detection of the malignant cells by using the data from the wave detector, and to arrange that the analyzer determines local differences of estimated stiffness values of the tissue under investigation, wherein the analyzer is arranged to use these local and/or asymmetrical differences of stiffness values as an indication for the presence or nonpresence of malignant cells. The above-mentioned local differences are considered to differentiate between diseased tissue and healthy tissue.

In some examples it is helpful that the data processing device comprises or connects to a data storage device for storage of data from the wave detector. This makes a comparison of current data with data stored in the data storage device possible, which can be helpful in determining differences in the collected data over time, which may also be an indication for the occurrence of malignant cells. It is then preferable that the analyzer is equipped to operate on data from the wave detector and/or from the data storage device, wherein the data has different timestamps so as to enable a longitudinal comparison of data over time and to derive from this comparison an indication of the presence of malignant cells.

A beneficial way of using the system of the disclosure is that the system is designed for processing data derived from a humans breast. In such a design the system and method of the invention may add crucial information to the information derived with a conventional mammography apparatus and method, or be used in addition to a conventional mammography apparatus and method, so as to increase the reliability of detecting malignant cells. The following disclosure is best understood as a system and method to investigate a humans breast, although the system and method can be applied more broadly, also for the detection of malignant cells in other tissue

The system comprises a displacement actuator for displacing a tissue engaging surface or contact area along a predetermined path and/or applying a predetermined load on the tissue to be investigated. This is an effective way of mechanically exciting the tissue so that the detector can be arranged to collect and provide data to the data processing device, said data relating at least to the tissue being disengaged and released from a load by the displacement actuator, and to the tissue being engaged and loaded by the displacement actuator (in the complete path up) to a predetermined full load. Comparing the data gathered from the tissue in the non-loaded situation with the data from the tissue in the loaded situation, and preferably also in the path between the unloaded and the loaded situation, can then be used to derive an indication of the presence of malignant cells. For clarity it is further mentioned that the displacement actuator can be a static actuator, but also a dynamic actuator in the sense that the displacement actuator produces vibrations in addition to moving from disengagement until engagement of the tissue to be investigated.

To make processing of the data, in particular position data of the tissue engaging surface or contact area of the displacement actuator more easy, the displacement actuator is equipped with at least one marker which enables position detection of the tissue engaging surface or contact area. Detection of the marker is then easy by arranging that the detector is equipped for detection of electromagnetic waves, which may be visual or nonvisual waves. It may be advantageous to provide a further detectable marker on the supporting surface against which the displacement actuator presses the tissue under investigation.

In some examples it is useful that the data collected and provided by the wave detector to the data processing device relate to a complete load-path-trajectory wherein the load provided by the displacement actuator on the tissue develops from no-load to the predetermined full load. The accuracy and reliability of the detection of malignant cells can thus be increased.

Surprisingly the inventors have found that it may already suffice for the detection of malignant cells that the data processing device is equipped to estimate a contact area between the displacement actuator and the tissue during engagement of the tissue by the displacement actuator. The extent of this contact area can already be seen as an indirect measure for the tissue stiffness.

Further, stiffness differences as such are found to be strongly indicative for the existence or occurrence of malignant cells in an individual case. When looking at a population, evidence exists that stiffness as such can be used as a biomarker, comparable with mammographic density. The inventors remark that the identification of high but symmetrical outcome results can be used in a population-based stratification of risk of developing malignant cells and can serve as a biomarker in risk-based screening approaches.

More reliable results than with estimation can be achieved when the displacement actuator is equipped with a capacitive measurement organ to determine the contact area between the displacement actuator and the tissue during engagement of the tissue by the displacement actuator.

Preferably the analyzer is arranged to derive an estimated stiffness value of the tissue from the estimated or measured contact area between the displacement actuator and the tissue during engagement of the tissue by the displacement actuator, or from a longitudinal comparison of the estimated or measured contact area developing over time and to derive from this comparison an indication of the presence of malignant cells.

Best results may be achieved when the estimation or measurement of the contact area between the displacement actuator and the tissue is monitored during a complete cycle of engaging and disengaging of the tissue by the displacement actuator.

In more advanced examples it is preferable that the analyzer is arranged to derive an estimated stiffness value of the tissue from a measured or calculated force applied to the tissue by the displacement actuator and the estimated or measured contact area between the displacement actuator and the tissue during engagement of the tissue by the displacement actuator.

In even further advanced examples it is preferable that the analyzer is arranged to derive an estimated stiffness value of the tissue from a longitudinal comparison of a measured or calculated force applied to the tissue by the displacement actuator over time, and the estimated or measured contact area developing over time between the displacement actuator and the tissue during engagement of the tissue by the displacement actuator.

There are examples in which the data processing device is equipped to determine from the data an estimated volume of the tissue being investigated. It may then or in other embodiments be preferable that the data processing device is equipped to determine from the data an estimated volume of the tissue being investigated when the displacement actuator is inactive and/or when the displacement actuator applies a load on the tissue and/or during a complete cycle of engaging and disengaging of the tissue by the displacement actuator.

Suitably the analyzer is arranged to derive an estimated stiffness value of the tissue from a measured or calculated force applied to the tissue and at least one of the estimated volume of the tissue, the contact area between the displacement actuator and the tissue, and a projected area obtained from a projection of the volume of the tissue, one thing and another derived from the data when the displacement actuator is inactive and/or when the displacement actuator applies a constant load to the tissue, and/or from the data when the displacement actuator applies an increasing load on the tissue. The term 'projected area' as used herein refers to a virtual contour projected in a line of sight from the detector to a supporting surface against which the displacement actuator presses the tissue under investigation.

It is further preferred that the system is equipped with a position sensor for measuring a position of a tissue engaging surface of the displacement actuator and/or a force sensor for measuring a force that the displacement actuator provides to the tissue, which position sensor and/or force sensor are connected to the data processing device for use by the data processing device in combination with the estimated or measured contact area between the displacement actuator and the tissue during engagement of the tissue by the displacement actuator so as to arrange that the load applied by the displacement actuator to the tissue is normalized to a pressure which the displacement actuator applies to the tissue.

Already mentioned above is a feasible option that the system is designed for processing data derived from a humans breast. It may then in particular be preferred that the system is designed for processing data derived from a left breast and a right breast from a same patient, and to arrange that the analyzer derives from the collected data certain differences between the left breast and the right breast which may support an indication of malignant cells in one breast or both breasts.

The invention will hereinafter be further elucidated with reference to the drawing of an exemplary embodiment of a system.

### Brief Description of the Figures

In the drawings:
- Figure 1 shows a basic layout of a system according to the present disclosure;
- Figure 2 shows a top view at the system of figure 1 during investigation; and
- Figure 3 shows a top view at the system of figure 1 during investigation of slightly deformed tissue caused by the presence of malignant cells.

Whenever in the figures the same reference numerals are applied, these numerals refer to the same parts.

### Detailed Description

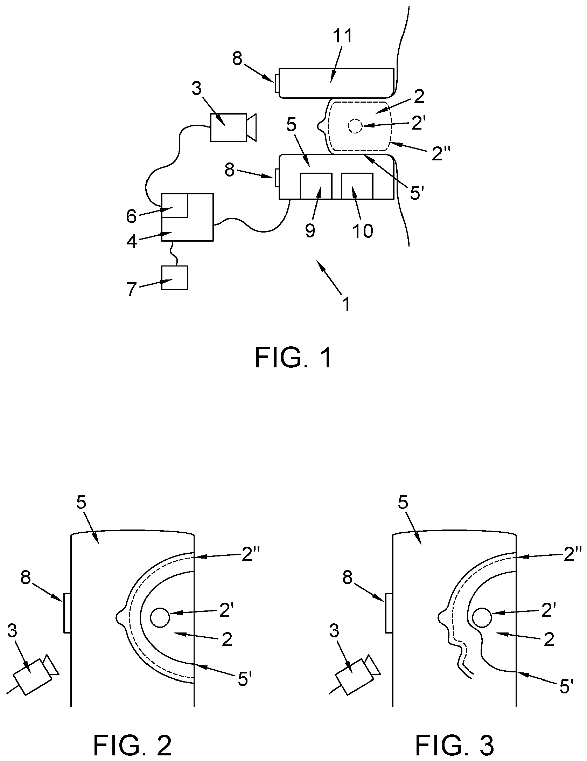

Figure 1 shows a basic layout of a system 1 of the invention for screening tissue 2 on the presence of malignant cells in said tissue 2. In the shown case the tissue is of a breast, although this is not essential. Instead of a breast also other tissue may be screened with the system and method of the invention on the occurrence of malignant cells.

**The** system 1 comprises a wave detector 3 and a data processing device 4 connected or connectable to the wave detector 3 for processing data received from the wave detector 3.

**The** system 1 further comprises an actuator 5 to mechanically excite the tissue 2 which is suspected to comprise malignant cells, and the processing device 4 comprises an analyzer 6 connected to the wave detector 3 for analyzing the data received from the wave detector 3 in response to the actuator 5 mechanically exciting the tissue 2. The analyzer 6 is arranged to identify and select the tissue 2' which has in comparison with tissue 2" that is not suspected to comprise malignant cells, an elevated probability to comprise malignant cells.

The choice of wave detector 3 that is used is dependent on the type of actuator 5 that is applied to mechanically excite the tissue 2. Mechanically exciting the tissue 2 may be done for instance with a sound emitter, an ultrasound emitter, a pulsed laser source (which is known to inflict ultrasound waves in tissue subjected to the pulsed laser source), or - as is shown in figure 1 - with a mechanical displacement actuator 5. Figure 1 also shows a supporting surface 11 against which the displacement actuator 5 presses the tissue 2 under investigation.

Corresponding to the type of actuator used, the wave detector 3 may be selected as one of an (ultra-) sound wave detector, a visual light spectrum 2D or 3D static picture camera, a visual light spectrum 2D or 3D moving picture camera, or even an infrared detector, an ultraviolet detector, a LIDAR, a radar, or a microwave antenna. When as is shown in figure 1, a camera is applied this preferably is a CCD-camera 3.

When the actuator is a displacement actuator 5 it must be arranged for displacing a tissue engaging surface or contact area 5' of the displacement actuator 5 along a predetermined path and/or applying a predetermined load on the tissue 2 to be investigated. The resulting deformation of the tissue 2 can then be observed with the wave detector 3. Based thereon the analyzer 6 of the data processing device 4 determines from the data received from the wave detector 3 an estimated stiffness value of the tissue 2 under investigation, wherein the analyzer 6 is arranged to use this stiffness value as an indication for the presence or nonpresence of malignant cells.

A nonlimiting example that relates to the envisaged detection of malignant cells with the wave detector 3 is the following. For comparative purposes figure 3 differs from figure 2 to show that tissue 2 under investigation may behave differently depending on the location and nature of the presence of malignant cells 2' in the tissue 2. In figure 2 the pressure applied to the tissue 2 homogeneously spreads through the tissue 2 under investigation, whereas in figure 3 there is an inhomogeneous spreading of the pressure applied to the tissue 2. This difference may serve as a visual indication which is detectable by the wave detector 3 that further investigation may be warranted as to the (non-)presence of malignant cells.

In a refined embodiment of the system 1 of the invention the analyzer 6 determines from the data received from the wave detector 3 local differences of estimated stiffness values of the tissue 2 under investigation, wherein the analyzer 6 is arranged to use these local differences of stiffness values as an indication for the presence or nonpresence of malignant cells. The local differences in stiffness value correspond to healthy tissue and tissue comprising malignant cells. The deformation of the tissue as shown in figure 3 is an indication of such local differences in stiffness value.

Figure 1 further shows that the data processing device 4 comprises or connects to a data storage device 7 for storage of data received from the wave detector 3. This enables that the analyzer 6 can be equipped to operate on data received from the wave detector 3 and/or from the data storage device 7, wherein the data has different timestamps so as to enable a longitudinal comparison of data over time and to derive from this comparison an indication of the probable presence of malignant cells.

Figure 1 depicts the situation that the tissue 2 under investigation is mechanically excited by a load applied by the displacement actuator 5 on the tissue 2. It is preferable however that the wave detector 3 is enabled to collect and provide data to the data processing device 4 from two different situations, notably one situation in which said data relates at least to the tissue 2 being disengaged and released from a load by the displacement actuator 5, and another situation in which the tissue 2 is being engaged and loaded by the displacement actuator 5, the latter preferably according to a predetermined trajectory until reaching a predetermined full load being applied to the tissue 2.

Figure 1 further schematically shows that the displacement actuator 5 is equipped with at least one marker 8 which enables position detection of the tissue engaging surface or contact area 5' of the displacement actuator 5. The tissue engaging surface or contact area 5' is also shown in the top view provided by figure 2. For the purpose of position detection of the tissue engaging surface or contact area 5' it is preferred that the wave detector 3 is arranged to detect the position of the marker 8. Such a detectable marker 8 is preferably also provided on the supporting surface 11 against which the displacement actuator 5 presses the tissue 2 under investigation. This is shown in fig. 1.

Most preferably the data collected and provided by the wave detector 3 to the data processing device 4 relates to a complete load-path-trajectory wherein the load provided by the displacement actuator 5 on the tissue 2 develops from no-load to the predetermined full load.

In certain embodiments it is useful that the displacement actuator 5 is equipped with a capacitive measurement organ (not shown) to determine the contact area 5' (see figure 2) between the displacement actuator 5 and the tissue 2 during engagement of the tissue 2 by the displacement actuator 5. If such a capacitive measurement organ is not applied, the data processing device 4 can be equipped to estimate from the data the contact area between the displacement actuator 5 and the tissue 2 as it develops during engagement of the tissue 2 by the displacement actuator 5. In particular it may then be desirable that the analyzer 6 is arranged to derive an estimated stiffness value of the tissue 2 from the estimated or measured contact area 5' between the displacement actuator 5 and the tissue 2 during engagement of the tissue 2 by the displacement actuator 5, or from a longitudinal comparison of the estimated or measured contact area 5' developing over time and to derive from this comparison an indication of the presence of malignant cells. The reliability of the detection of malignant cells may be further improved by arranging that the estimation or measurement of the contact area 5' between the displacement actuator 5 and the tissue 2 is monitored during a complete cycle of engaging and disengaging of the tissue 2 by the displacement actuator 5.

In another embodiment the analyzer 6 is arranged to derive an estimated stiffness value of the tissue 2 from a measured or calculated force applied to the tissue 2 by the displacement actuator 5 and the estimated or measured contact area 5' between the displacement actuator 5 and the tissue 2 during engagement of the tissue 2 by the displacement actuator 5. More preferred is that the analyzer 6 is arranged to derive an estimated stiffness value of the tissue 2 from a longitudinal comparison of a measured or calculated force applied to the tissue 2 by the displacement actuator 5 over time, and the estimated or measured contact area 5' developing over time between the displacement actuator 5 and the tissue 2 during engagement of the tissue 2 by the displacement actuator 5. In combination the force applied by the displacement actuator 5 and the estimated or measured contact area 5', enables the calculation of an estimated or measured pressure applied to the tissue 2 which is a factor which can be taken into account in the estimation whether or not malignant cells are present in the tissue 2.

In still another embodiment the data processing device is equipped to determine from the data an estimated volume of the tissue being investigated. In this embodiment it is preferred that the data processing device 4 is equipped to determine from the data an estimated volume of the tissue 2 being investigated when the displacement actuator 5 is inactive and/or when the displacement actuator 5 applies a load on the tissue 2 and/or during a complete cycle of engaging and disengaging of the tissue 2 by the displacement actuator 5.

It is also possible to combine data and to arrange the analyzer 6 to derive an estimated stiffness value of the tissue 2 from a measured or calculated force applied to the tissue 2 and at least one of the estimated volume of the tissue 2, the contact area 5' between the displacement actuator 5 and the tissue 2, and a projected area obtained from a projection of the volume of the tissue, one thing and another derived from the data when the displacement actuator 5 is inactive and/or when the displacement actuator 5 applies a constant load to the tissue 2, and/or from the data when the displacement actuator 5 applies an increasing load on the tissue 2. The term 'projected area' as used herein refers to a virtual contour projected in a line of sight from the detector to a supporting surface 11 against which the displacement actuator 5 presses the tissue 2 under investigation.

**To** improve accuracy the system 1 may be equipped with a position sensor 9 for measuring a position of a tissue engaging surface 5' of the displacement actuator 5 and/or a force sensor 10 for measuring a force that the displacement actuator 5 provides to the tissue 2, which position sensor 9 and/or force sensor 10 are connected (not shown) to the data processing device 4 for use by the data processing device 4 in combination with the estimated or measured contact area 5' between the displacement actuator 5 and the tissue 2 during engagement of the tissue 2 by the displacement actuator 5 so as to arrange that the load applied by the displacement actuator 5 to the tissue 2 is normalized to a pressure which the displacement actuator 5 applies to the tissue 2.

Although this is not essential to the invention, figure 1 shows that the system 1 processes data derived from a humans breast. Under circumstances it may be preferable that the system 1 is designed for processing data derived from a left breast and a right breast from a same patient, and to arrange that the analyzer 6 derives from differences between the left breast and the right breast an indication of malignant cells in one breast or in both breasts.

Embodiments of the present invention can include every combination of features that are disclosed herein independently from each other. Although the invention has been discussed in the foregoing with reference to an exemplary embodiment of the system and method of the invention, the invention is not restricted to this particular embodiment which can be varied in many ways without departing from the invention. The discussed exemplary embodiment shall therefore not be used to construe the appended claims strictly in accordance therewith. On the contrary the embodiment is merely intended to explain the wording of the appended claims without intent to limit the claims to this exemplary embodiment. The scope of protection of the invention shall therefore be construed in accordance with the appended claims only, wherein a possible ambiguity in the wording of the claims shall be resolved using this exemplary embodiment.

Variations and modifications of the present invention will be obvious to those skilled in the art and it is intended to cover in the appended claims all such modifications and equivalents. The entire disclosures of all references, applications, patents, and publications cited above are hereby incorporated by reference. Unless specifically stated as being "essential" above, none of the various components or the interrelationship thereof are essential to the operation of the invention. Rather, desirable results can be achieved by substituting various components and/or reconfiguration of their relationships with one another.

Optionally, embodiments of the present invention can include a general or specific purpose computer or distributed system programmed with computer software implementing steps described above, which computer software may be in any appropriate computer language, including but not limited to C++, FORTRAN, ALGOL, BASIC, Java, Python, Linux, assembly language, microcode, distributed programming languages, etc. The system may also include a plurality of such computers / distributed systems (e.g., connected over the Internet and/or one or more intranets) in a variety of hardware implementations. For example, data processing can be performed by an appropriately programmed microprocessor, computing cloud, Application Specific Integrated Circuit (ASIC), Field Programmable Gate Array (FPGA), or the like, in conjunction with appropriate memory, network, and bus elements. One or more processors and/or microcontrollers can operate via instructions of the computer code and the software is preferably stored on one or more tangible non-transitive memory-storage devices.

## Claims

1. A system (1) for screening tissue (2) on the presence of malignant cells in said tissue (2), which system (1) comprises a wave detector (3) and a data processing device (4) connected or connectable to the wave detector (3) for processing data received from the wave detector (3), wherein, the system (1) comprises an actuator (5) to mechanically excite the tissue (2) which is suspected to comprise malignant cells, and the data processing device (4) comprises an analyzer (6) connected to the wave detector (3) for analyzing the data received from the wave detector (3) in response to the actuator (5) mechanically exciting the tissue (2), which analyzer (6) is arranged to identify the tissue (2') with an elevated probability to comprise malignant cells in comparison with tissue (2") that is not suspected to comprise malignant cells,
wherein the actuator is a displacement actuator (5) for displacing a tissue engaging surface (5') along a predetermined path and/or applying a predetermined load on the tissue (2) to be investigated and wherein the displacement actuator is equipped with at least one marker (8) which enables position detection of the tissue engaging surface or contact area (5') of the displacement actuator (5), with the wave detector (3) being adapted to detect the position of the marker (8).

2. The system of claim 1, wherein the analyzer (6) determines from the data received from the wave detector (3) an estimated stiffness value of the tissue (2) under investigation, wherein the analyzer (6) is arranged to use this stiffness value as an indication for the presence or nonpresence of malignant cells,
wherein optionally the analyzer (6) determines from the data received from the wave detector (3) local differences of estimated stiffness values of the tissue (2) under investigation, wherein the analyzer (6) is arranged to use these local differences of stiffness values as an indication for the presence or nonpresence of malignant cells.

3. The system of any one of claims 1 - 2, wherein:
the data processing device (4) comprises or connects to a data storage device (7) for storage of data received from the wave detector (3), and/or
the analyzer (6) is equipped to operate on data received from the wave detector (3) and/or from the data storage device (7), wherein the data has different timestamps so as to enable a longitudinal comparison of data over time and to derive from this comparison an indication of the probable presence of malignant cells; and/or
the actuator (5) is one of a sound emitter, an ultrasound emitter, a pulsed laser source, a displacement actuator (5); and/or
the data processing device (4) is equipped to determine from the data an estimated volume of the tissue (2) being investigated.

4. The system of any one of claims 1 - 3, wherein the wave detector (3) is arranged to collect and provide data to the data processing device (4), said data relating at least to the tissue (2) being disengaged and released from a load by the displacement actuator (5), and to the tissue (2) being engaged and loaded by the displacement actuator (5) to a predetermined full load.

5. The system of any one of claims 1 - 4, wherein the wave detector (3) is at least one of an (ultra-) sound wave detector, a visual light spectrum 2D or 3D static picture camera, a visual light spectrum 2D or 3D moving picture camera, an infrared detector, an ultraviolet detector, a LIDAR, a radar, a microwave antenna,
wherein optionally, the data collected and provided by the wave detector (3) to the data processing device (4) relates to a complete load-path-trajectory wherein the load provided by the displacement actuator (5) on the tissue (2) develops from no-load to the predetermined full load,
wherein optionally the camera (3) is a CCD-camera.

6. The system of any one of claims 1 - 5, wherein a further detectable marker (8) is provided on the supporting surface (11) against which the displacement actuator (5) presses the tissue (2) under investigation.

7. The system of any of claims 1 to 6, wherein the wave detector (3) is arranged to detect the position of the marker (8).

8. The system of any one of claims 1-7, wherein the data processing device (4) is equipped to estimate from the data a contact area (5') between the displacement actuator (5) and the tissue (2) during engagement of the tissue (2) by the displacement actuator (5).

9. The system of any one of claims 1 - 8, wherein the displacement actuator (5) is equipped with a capacitive measurement organ to determine a contact area (5') between the displacement actuator (5) and the tissue (2) during engagement of the tissue (2) by the displacement actuator (5).

10. The system of claim 8 or 9, wherein the analyzer (6) is arranged to derive an estimated stiffness value of the tissue from the estimated or measured contact area (5') between the displacement actuator (5) and the tissue (2) during engagement of the tissue (2) by the displacement actuator (5), or from a longitudinal comparison of the estimated or measured contact area (5') developing over time and to derive from this comparison an indication of the presence of malignant cells.

11. The system of any one of claims 8 - 10, wherein the estimation or measurement of the contact area (5') between the displacement actuator (5) and the tissue (2) is monitored during a complete cycle of engaging and disengaging of the tissue (2) by the displacement actuator (5).

12. The system of any one of claims 1 - 11, wherein the analyzer (6) is arranged to derive an estimated stiffness value of the tissue (2) from a measured or calculated force applied to the tissue (2) by the displacement actuator (5) and the estimated or measured contact area (5') between the displacement actuator (5) and the tissue (2) during engagement of the tissue (2) by the displacement actuator (5).

13. The system of any one of claims 1 - 12, wherein:
the analyzer (6) is arranged to derive an estimated stiffness value of the tissue (2) from a longitudinal comparison of a measured or calculated force applied to the tissue (2) by the displacement actuator (5) over time, and the estimated or measured contact area (5') developing over time between the displacement actuator (5) and the tissue (2) during engagement of the tissue (2) by the displacement actuator (5), and/or
the data processing device (4) is equipped to determine from the data an estimated volume of the tissue (2) being investigated when the displacement actuator (5) is inactive and/or when the displacement actuator (5) applies a load on the tissue (2) and/or during a complete cycle of engaging 5 and disengaging of the tissue (2) by the displacement actuator (5).

14. The system of any one of claims 8 - 13, wherein the analyzer (6) is arranged to derive an estimated stiffness value of the tissue (2) from a measured or calculated force applied to the tissue (2) and at least one of the estimated volume of the tissue (2), the contact area (5') between the displacement actuator (5) and the tissue (2), and a projected area obtained from a projection of the volume of the tissue (2), one thing and another derived from the data when the displacement actuator (5) is inactive and/or when the displacement actuator (5) applies a constant load to the tissue (2), and/or from the data when the displacement actuator (5) applies an increasing load on the tissue (2).

15. The system of any one of claims 1 - 14, wherein the system (1) is equipped with a position sensor (9) for measuring a position of a tissue engaging surface or contact area (5') of the displacement actuator (5) and/or a force sensor (10) for measuring a force that the displacement actuator (5) provides to the tissue (2), which position sensor(9) and/or force sensor (10) are connected to the data processing device (4) for use by the data processing device (4) in combination with the estimated or measured contact area (5') between the displacement actuator (5) and the tissue (2) during engagement of the tissue (2) by the displacement actuator (5) so as to arrange that the load applied by the displacement actuator (5) to the tissue (2) is normalized to a pressure which the displacement actuator (5) applies to the tissue (2).

## Patentansprüche

1. System (1) zum Screenen von Gewebe (2) auf das Vorhandensein von malignen Zellen in dem Gewebe (2), wobei das System (1) einen Wellendetektor (3) und eine Datenverarbeitungsvorrichtung (4) umfasst, die mit dem Wellendetektor (3) verbunden oder verbindbar ist, um von dem Wellendetektor (3) empfangene Daten zu verarbeiten, wobei das System (1) einen Aktuator (5) umfasst, um das Gewebe (2), das in Verdacht steht, maligne Zellen zu umfassen, mechanisch anzuregen, und die Datenverarbeitungsvorrichtung (4) einen Analysator (6) umfasst, der mit dem Wellendetektor (3) verbunden ist, um die von dem Wellendetektor (3) empfangenen Daten als Reaktion darauf zu analysieren, dass der Aktuator (5) das Gewebe (2) mechanisch anregt, wobei der Analysator (6) dazu ausgelegt ist, das Gewebe (2') mit einer erhöhten Wahrscheinlichkeit, maligne Zellen zu umfassen, im Vergleich zu Gewebe (2"), das nicht im Verdacht steht, maligne Zellen zu umfassen, zu identifizieren,
wobei der Aktuator ein Verschiebungsaktuator (5) zum Verschieben einer Gewebeeingriffsoberfläche (5') entlang eines vorbestimmten Pfads und/oder zum Aufbringen einer vorbestimmten Last auf das zu untersuchende Gewebe (2) ist und wobei der Verschiebungsaktuator mit mindestens einer Markierung (8) ausgestattet ist, die eine Positionserkennung der Gewebeeingriffsoberfläche oder des Kontaktbereichs (5') des Verschiebungsaktuators (5) ermöglicht, wobei der Wellendetektor (3) dazu ausgelegt ist, die Position der Markierung (8) zu erkennen.

2. System nach Anspruch 1, wobei der Analysator (6) aus den von dem Wellendetektor (3) empfangenen Daten einen geschätzten Steifigkeitswert des Gewebes (2), das Gegenstand der Untersuchung ist, bestimmt, wobei der Analysator (6) dazu ausgelegt ist, diesen Steifigkeitswert als einen Hinweis auf das Vorhandensein oder Nichtvorhandensein von malignen Zellen zu verwenden,
wobei optional der Analysator (6) aus den von dem Wellendetektor (3) empfangenen Daten lokale Unterschiede geschätzter Steifigkeitswerte des Gewebes (2), das Gegenstand der Untersuchung ist, bestimmt, wobei der Analysator (6) dazu ausgelegt ist, diese lokalen Unterschiede von Steifigkeitswerten als einen Hinweis auf das Vorhandensein oder Nichtvorhandensein von malignen Zellen zu verwenden.

3. System nach einem der Ansprüche 1 - 2, wobei:
die Datenverarbeitungsvorrichtung (4) eine Datenspeichervorrichtung (7) zur Speicherung von vom Wellendetektor (3) empfangenen Daten umfasst oder mit dieser verbunden ist und/oder
der Analysator (6) dazu ausgestattet ist, an von dem Wellendetektor (3) und/oder von der Datenspeichervorrichtung (7) empfangenen Daten zu arbeiten, wobei die Daten unterschiedliche Zeitstempel aufweisen, um einen Längsvergleich von Daten im Zeitverlauf zu ermöglichen und aus diesem Vergleich einen Hinweis auf das wahrscheinliche Vorhandensein von malignen Zellen abzuleiten; und/oder
der Aktuator (5) eines von einem Schallemitter, einem Ultraschallemitter, einer gepulsten Laserquelle, einem Verschiebungsaktuator (5) ist; und/oder
die Datenverarbeitungsvorrichtung (4) dazu ausgestattet ist, aus den Daten ein geschätztes Volumen des Gewebes (2), das untersucht wird, zu bestimmen.

4. System nach einem der Ansprüche 1 - 3, wobei der Wellendetektor (3) dazu ausgelegt ist, Daten zu sammeln und für die Datenverarbeitungsvorrichtung (4) bereitzustellen, wobei sich die Daten mindestens darauf beziehen, dass das Gewebe (2) durch den Verschiebungsaktuator (5) außer Eingriff gebracht und von einer Last gelöst wird, und dass das Gewebe (2) durch den Verschiebungsaktuator (5) in Eingriff genommen und auf eine vorbestimmte Volllast belastet wird.

5. System nach einem der Ansprüche 1 - 4, wobei der Wellendetektor (3) mindestens eines von einem (Ultra-)Schallwellendetektor, einer 2D- oder 3D-Standbildkamera mit sichtbarem Lichtspektrum, einer 2D- oder 3D-Filmkamera mit sichtbarem Lichtspektrum, einem Infrarotdetektor, einem Ultraviolettdetektor, einem LIDAR, einem Radar, einer Mikrowellenantenne ist,
wobei sich die durch den Wellendetektor (3) gesammelten und für die Datenverarbeitungsvorrichtung (4) bereitgestellten Daten optional auf eine vollständige Last-Weg-Kurve beziehen, wobei sich die durch den Verschiebungsaktuator (5) für das Gewebe (2) bereitgestellte Last von lastfrei zu der vorbestimmten Volllast entwickelt,
wobei die Kamera (3) optional eine CCD-Kamera ist.

6. System nach einem der Ansprüche 1 - 5, wobei eine weitere detektierbare Markierung (8) auf der Stützoberfläche (11) bereitgestellt ist, gegen die der Verschiebungsaktuator (5) das Gewebe (2), das Gegenstand der Untersuchung ist, drückt.

7. System nach einem der Ansprüche 1 bis 6, wobei der Wellendetektor (3) dazu ausgelegt ist, die Position der Markierung (8) zu erkennen.

8. System nach einem der Ansprüche 1-7, wobei die Datenverarbeitungsvorrichtung (4) dazu ausgestattet ist, aus den Daten einen Kontaktbereich (5') zwischen dem Verschiebungsaktuator (5) und dem Gewebe (2) während der Ineingriffnahme des Gewebes (2) durch den Verschiebungsaktuator (5) zu schätzen.

9. System nach einem der Ansprüche 1 - 8, wobei der Verschiebungsaktuator (5) mit einem kapazitiven Messwerkzeug ausgestattet ist, um einen Kontaktbereich (5') zwischen dem Verschiebungsaktuator (5) und dem Gewebe (2) während der Ineingriffnahme des Gewebes (2) durch den Verschiebungsaktuator (5) zu bestimmen.

10. System nach Anspruch 8 oder 9, wobei der Analysator (6) dazu ausgelegt ist, einen geschätzten Steifigkeitswert des Gewebes aus dem geschätzten oder gemessenen Kontaktbereich (5') zwischen dem Verschiebungsaktuator (5) und dem Gewebe (2) während der Ineingriffnahme des Gewebes (2) durch den Verschiebungsaktuator (5) oder aus einem Längsvergleich des sich im Zeitverlauf entwickelnden geschätzten oder gemessenen Kontaktbereichs (5') abzuleiten und aus diesem Vergleich einen Hinweis auf das Vorhandensein von malignen Zellen abzuleiten.

11. System nach einem der Ansprüche 8 - 10, wobei die Schätzung oder Messung des Kontaktbereichs (5') zwischen dem Verschiebungsaktuator (5) und dem Gewebe (2) während eines vollständigen Zyklus des Ineingriffnehmens und Außereingriffbringens des Gewebes (2) durch den Verschiebungsaktuator (5) überwacht wird.

12. System nach einem der Ansprüche 1 - 11, wobei der Analysator (6) dazu ausgelegt ist, einen geschätzten Steifigkeitswert des Gewebes (2) aus einer gemessenen oder berechneten Kraft, die durch den Verschiebungsaktuator (5) auf das Gewebe (2) aufgebracht wird, und dem geschätzten oder gemessenen Kontaktbereich (5') zwischen dem Verschiebungsaktuator (5) und dem Gewebe (2) während der Ineingriffnahme des Gewebes (2) durch den Verschiebungsaktuator (5) abzuleiten.

13. System nach einem der Ansprüche 1 - 12, wobei:
der Analysator (6) dazu ausgelegt ist, einen geschätzten Steifigkeitswert des Gewebes (2) aus einem Längsvergleich einer gemessenen oder berechneten Kraft, die durch den Verschiebungsaktuator (5) auf das Gewebe (2) aufgebracht wird, im Zeitverlauf und dem geschätzten oder gemessenen Kontaktbereich (5'), der sich im Zeitverlauf zwischen dem Verschiebungsaktuator (5) und dem Gewebe (2) entwickelt, während der Ineingriffnahme des Gewebes (2) durch den Verschiebungsaktuator (5) abzuleiten, und/oder
die Datenverarbeitungsvorrichtung (4) dazu ausgestattet ist, aus den Daten ein geschätztes Volumen des Gewebes (2) zu bestimmen, das untersucht wird, wenn der Verschiebungsaktuator (5) inaktiv ist und/oder wenn der Verschiebungsaktuator (5) eine Last auf das Gewebe (2) aufbringt und/oder während eines vollständigen Zyklus des Ineingriffnehmens 5 und Außereingriffbringens des Gewebes (2) durch den Verschiebungsaktuator (5).

14. System nach einem der Ansprüche 8 - 13, wobei der Analysator (6) dazu ausgelegt ist, einen geschätzten Steifigkeitswert des Gewebes (2) aus einer gemessenen oder berechneten Kraft, die auf das Gewebe (2) aufgebracht wird, und mindestens einem von dem geschätzten Volumen des Gewebes (2), dem Kontaktbereich (5') zwischen dem Verschiebungsaktuator (5) und dem Gewebe (2) und einem projizierten Bereich, der aus einer Projektion des Volumens des Gewebes (2) erhalten wird, abzuleiten, wobei das eine und andere aus den Daten abgeleitet sind, wenn der Verschiebungsaktuator (5) inaktiv ist und/oder wenn der Verschiebungsaktuator (5) eine konstante Last auf das Gewebe (2) aufbringt, und/oder aus den Daten, wenn der Verschiebungsaktuator (5) eine zunehmende Last auf das Gewebe (2) aufbringt.

15. System nach einem der Ansprüche 1 - 14, wobei das System (1) mit einem Positionssensor (9) zum Messen einer Position einer Gewebeeingriffsoberfläche oder eines Kontaktbereichs (5') des Verschiebungsaktuators (5) und/oder einem Kraftsensor (10) zum Messen einer Kraft, die der Verschiebungsaktuator (5) dem Gewebe (2) bereitstellt, ausgestattet ist, wobei der Positionssensor (9) und/oder der Kraftsensor (10) mit der Datenverarbeitungsvorrichtung (4) zur Verwendung durch die Datenverarbeitungsvorrichtung (4) in Kombination mit dem geschätzten oder gemessenen Kontaktbereich (5') zwischen dem Verschiebungsaktuator (5) und dem Gewebe (2) während der Ineingriffnahme des Gewebes (2) durch den Verschiebungsaktuator (5) verbunden sind, um dafür zu sorgen, dass die durch den Verschiebungsaktuator (5) auf das Gewebe (2) aufgebrachte Last auf einen Druck normiert wird, den der Verschiebungsaktuator (5) auf das Gewebe (2) aufbringt.

## Revendications

1. Système (1) permettant le dépistage d'un tissu (2) pour détecter la présence de cellules malignes dans ledit tissu (2), lequel système (1) comprend un détecteur d'ondes (3) et un dispositif de traitement de données (4) connecté ou pouvant être connecté au détecteur d'ondes (3) pour traiter des données reçues du détecteur d'ondes (3), ledit système (1) comprenant un actionneur (5) pour exciter mécaniquement le tissu (2) qui est soupçonné de comprendre des cellules malignes, et le dispositif de traitement de données (4) comprend un analyseur (6) connecté au détecteur d'ondes (3) pour analyser les données reçues du détecteur d'ondes (3) en réponse à l'excitation mécanique du tissu (2) par l'actionneur (5), lequel analyseur (6) est agencé pour identifier le tissu (2') présentant une probabilité élevée de comprendre des cellules malignes par rapport au tissu (2") qui n'est pas soupçonné de comprendre des cellules malignes,
dans lequel l'actionneur est un actionneur de déplacement (5) permettant de déplacer une surface de mise en prise de tissu (5') le long d'un trajet prédéterminé et/ou d'appliquer une charge prédéterminée sur le tissu (2) à étudier et dans lequel l'actionneur de déplacement est équipé d'au moins un marqueur (8) qui permet la détection de position de la surface de mise en prise de tissu ou d'une zone de contact (5') de l'actionneur de déplacement (5), le détecteur d'ondes (3) étant adapté pour détecter la position du marqueur (8).

2. Système de la revendication 1, dans lequel l'analyseur (6) détermine à partir des données reçues du détecteur d'ondes (3) une valeur de rigidité estimée du tissu (2) étudié, dans lequel l'analyseur (6) est agencé pour utiliser cette valeur de rigidité comme indication de la présence ou de la non-présence de cellules malignes,
dans lequel, éventuellement, l'analyseur (6) détermine à partir des données reçues du détecteur d'ondes (3) des différences locales de valeurs de rigidité estimées du tissu (2) étudié, dans lequel l'analyseur (6) est agencé pour utiliser ces différences locales de valeurs de rigidité comme indication de la présence ou de la non-présence de cellules malignes.

3. Système de l'une quelconque des revendications 1 à 2, dans lequel :
le dispositif de traitement de données (4) comprend ou se connecte à un dispositif de stockage de données (7) pour stocker des données reçues du détecteur d'ondes (3), et/ou
l'analyseur (6) est équipé pour exploiter des données reçues du détecteur d'ondes (3) et/ou du dispositif de stockage de données (7), dans lequel les données présentent des horodatages différents afin de permettre une comparaison longitudinale de données au fil du temps et de dériver de cette comparaison une indication de la présence probable de cellules malignes ; et/ou
l'actionneur (5) est l'un d'un émetteur de son, d'un émetteur d'ultrasons, d'une source laser pulsée, d'un actionneur de déplacement (5) ; et/ou
le dispositif de traitement de données (4) est équipé pour déterminer à partir des données un volume estimé du tissu (2) étudié.

4. Système de l'une quelconque des revendications 1 à 3, dans lequel le détecteur d'ondes (3) est agencé pour collecter et fournir des données au dispositif de traitement de données (4), lesdites données se rapportant au moins au fait que le tissu (2) est désengagé et libéré d'une charge par l'actionneur de déplacement (5), et au fait que le tissu (2) est en prise et chargé par l'actionneur de déplacement (5) à une pleine charge prédéterminée.

5. Système de l'une quelconque des revendications 1 à 4, dans lequel le détecteur d'ondes (3) est au moins l'un d'un détecteur d'ondes (ultra)sonores, d'une caméra d'images statiques 2D ou 3D dans le spectre de lumière visible, d'une caméra d'images animées 2D ou 3D dans le spectre de lumière visible, d'un détecteur infrarouge, d'un détecteur ultraviolet, d'un LIDAR, d'un radar, d'une antenne à micro-ondes,
dans lequel, éventuellement, les données collectées et fournies par le détecteur d'ondes (3) au dispositif de traitement de données (4) se rapportent à une trajectoire complète de trajet de charge dans laquelle la charge fournie par l'actionneur de déplacement (5) sur le tissu (2) passe d'une charge nulle à la pleine charge prédéterminée,
dans lequel, éventuellement, la caméra (3) est une caméra CCD.

6. Système de l'une quelconque des revendications 1 à 5, dans lequel un marqueur détectable supplémentaire (8) est prévu sur la surface de support (11) contre laquelle l'actionneur de déplacement (5) presse le tissu (2) étudié.

7. Système de l'une quelconque des revendications 1 à 6, dans lequel le détecteur d'ondes (3) est agencé pour détecter la position du marqueur (8).

8. Système de l'une quelconque des revendications 1 à 7, dans lequel le dispositif de traitement de données (4) est équipé pour estimer à partir des données une zone de contact (5') entre l'actionneur de déplacement (5) et le tissu (2) pendant la mise en prise du tissu (2) par l'actionneur de déplacement (5).

9. Système de l'une quelconque des revendications 1 à 8, dans lequel l'actionneur de déplacement (5) est équipé d'un organe de mesure capacitif pour déterminer une zone de contact (5') entre l'actionneur de déplacement (5) et le tissu (2) pendant la mise en prise du tissu (2) par l'actionneur de déplacement (5).

10. Système de l'une des revendications 8 ou 9, dans lequel l'analyseur (6) est agencé pour dériver une valeur de rigidité estimée du tissu à partir de la zone de contact estimée ou mesurée (5') entre l'actionneur de déplacement (5) et le tissu (2) pendant la mise en prise du tissu (2) par l'actionneur de déplacement (5), ou à partir d'une comparaison longitudinale de la zone de contact estimée ou mesurée (5') se développant au fil du temps, et pour dériver de cette comparaison une indication de la présence de cellules malignes.

11. Système de l'une quelconque des revendications 8 à 10, dans lequel l'estimation ou la mesure de la zone de contact (5') entre l'actionneur de déplacement (5) et le tissu (2) est surveillée pendant un cycle complet de mise en prise et de désengagement du tissu (2) par l'actionneur de déplacement (5).

12. Système de l'une quelconque des revendications 1 à 11, dans lequel l'analyseur (6) est agencé pour dériver une valeur de rigidité estimée du tissu (2) à partir d'une force mesurée ou calculée appliquée au tissu (2) par l'actionneur de déplacement (5) et de la zone de contact estimée ou mesurée (5') entre l'actionneur de déplacement (5) et le tissu (2) pendant la mise en prise du tissu (2) par l'actionneur de déplacement (5).

13. Système de l'une quelconque des revendications 1 à 12, dans lequel :
l'analyseur (6) est agencé pour dériver une valeur de rigidité estimée du tissu (2) à partir d'une comparaison longitudinale d'une force mesurée ou calculée appliquée au tissu (2) par l'actionneur de déplacement (5) au fil du temps, et de la zone de contact estimée ou mesurée (5') se développant au fil du temps entre l'actionneur de déplacement (5) et le tissu (2) pendant la mise en prise du tissu (2) par l'actionneur de déplacement (5), et/ou
le dispositif de traitement de données (4) est équipé pour déterminer à partir des données un volume estimé du tissu (2) étudié lorsque l'actionneur de déplacement (5) est inactif et/ou lorsque l'actionneur de déplacement (5) applique une charge sur le tissu (2) et/ou pendant un cycle complet de mise en prise 5 et de désengagement du tissu (2) par l'actionneur de déplacement (5).

14. Système de l'une quelconque des revendications 8 à 13, dans lequel l'analyseur (6) est agencé pour dériver une valeur de rigidité estimée du tissu (2) à partir d'une force mesurée ou calculée appliquée au tissu (2) et d'au moins l'un parmi le volume estimé du tissu (2), la zone de contact (5') entre l'actionneur de déplacement (5) et le tissu (2) et une zone projetée obtenue à partir d'une projection du volume du tissu (2), d'une chose et d'une autre dérivées des données lorsque l'actionneur de déplacement (5) est inactif et/ou lorsque l'actionneur de déplacement (5) applique une charge constante au tissu (2), et/ou à partir des données lorsque l'actionneur de déplacement (5) applique une charge croissante sur le tissu (2).

15. Système de l'une quelconque des revendications 1 à 14, dans lequel le système (1) est équipé d'un capteur de position (9) pour mesurer une position d'une surface de mise en prise de tissu ou d'une zone de contact (5') de l'actionneur de déplacement (5) et/ou d'un capteur de force (10) pour mesurer une force que l'actionneur de déplacement (5) fournit au tissu (2), le(s)quel(s) capteur de position (9) et/ou capteur de force (10) est/sont connecté(s) au dispositif de traitement de données (4) pour une utilisation par le dispositif de traitement de données (4) en combinaison avec la zone de contact estimée ou mesurée (5') entre l'actionneur de déplacement (5) et le tissu (2) pendant la mise en prise du tissu (2) par l'actionneur de déplacement (5) afin que la charge appliquée par l'actionneur de déplacement (5) au tissu (2) soit normalisée à une pression que l'actionneur de déplacement (5) applique au tissu (2).
